# EUROPÄISCHE PATENTANMELDUNG

(11) **EP 4 623 940 A1**
(43) Veröffentlichungstag der Anmeldung: **01.10.2025**
(21) Anmeldenummer: 24167630.3
(22) Anmeldetag: 28.03.2024
(51) Int. Cl.: A61L 9/12, B60H 3/00

(54) **BEDUFTUNGSVORRICHTUNG FÜR EIN FAHRZEUG**

(71) Anmelder: Supair-Tel AG, 8152 Glattbrugg (CH)
(72) Erfinder: Guggenheim, Rudolf Heinrich, 8832 Wollerau (CH)
(74) Vertreter: E. Blum & Co. AG

(57) **Zusammenfassung**

Die Erfindung betrifft eine Beduftungsvorrichtung für ein Fahrzeug mit einem Abdeckelement (11), einer Haltevorrichtung (12) und einem Duftstoffbehälter (14). Die Haltevorrichtung (12) dient dem Anbringen der Beduftungsvorrichtung (10) im Innenraum des Fahrzeuges, wobei die Haltevorrichtung (12) an der Rückseite (11b) des Abdeckelements (11) befestigt ist. Der Duftstoffbehälter (14) ist rückseitig zum Abdeckelement (11) angeordnet und an der Haltevorrichtung (12) befestigt. Die Vorderseite (11a) des Abdeckelements (11) weist eine grössere Fläche auf, als eine Aussenfläche (14a) des Duftstoffbehälters (14), welche gegen die Rückseite (11b) des Abdeckelements (11) gerichtet ist. Dadurch wird der Duftstoffbehälter (14) vom Abdeckelement (11) im Fahrzeug diskret verdeckt.

## Beschreibung

### Gebiet der Erfindung

Die Erfindung betrifft eine Beduftungsvorrichtung für ein Fahrzeug. Die Beduftungsvorrichtung umfasst ein Abdeckelement, eine Haltevorrichtung und einen Duftstoffbehälter. Das Abdeckelement weist eine Vorderseite und eine Rückseite auf. Die Haltevorrichtung ist ausgestaltet, die Beduftungsvorrichtung im Innenraum des Fahrzeuges anzubringen. Die Haltevorrichtung ist an der Rückseite des Abdeckelements befestigt. Der Duftstoffbehälter ist rückseitig zum Abdeckelement angeordnet, wobei der Duftstoffbehälter am Abdeckelement und/oder an der Haltevorrichtung befestigt ist.

Im Weiteren betrifft die Erfindung ein Fahrzeug mit einem Innenraum, in welchem eine Paneele mit mindestens zwei Displays angeordnet ist und die Beduftungsvorrichtung an der Paneele montiert ist.

### Hintergrund

Es sind Beduftungsvorrichtungen bekannt, welche im Fahrzeuginneren aufgehängt werden können und z.B. ein mit Duftstoff getränktes Material aufweisen. Solche Vorrichtungen sind kostengünstig herstellbar. Ist die Beduftungsvorrichtung am Innenrückspiegel aufgehängt, kann die Sicht des Fahrers beeinträchtigt werden.

Im Weiteren sind Behälter mit Duftstoff bekannt, welche an einem Lüftungsgitter im Innenraum eines Fahrzeuges befestigt werden. Der Behälter für den Duftstoff weist Einlassöffnungen auf, sodass Luft aus der Lüftung in den Behälter hineinströmt, mit dem Duftstoff in Kontakt gerät und angereichert mit Duftmolekülen den Behälter durch Auslassöffnungen verlässt. Solche Behälter können mittels eines Klemmteils am Lüftungsgitter befestigt und von diesem auch wieder entfernt werden.

### Darstellung der Erfindung

Es stellt sich die Aufgabe, eine Beduftungsvorrichtung mit einem Duftstoffbehälter bereitzustellen, wobei der Duftstoffbehälter möglichst unauffällig im Innenraum eines Fahrzeugs angeordnet werden kann.

Die Aufgabe wird durch eine Beduftungsvorrichtung mit den Merkmalen des Anspruchs 1 gelöst. Die erfindungsgemäss Beduftungsvorrichtung zur Montage in einem Fahrzeug geeignet. Der Begriff "Fahrzeug" umfasst Landfahrzeuge, wie Personenkraftwagen und Lastkraftwagen, und auch Luftfahrzeuge.

Die Beduftungsvorrichtung umfasst
- ein Abdeckelement mit einer Vorderseite und einer Rückseite.
- eine Haltevorrichtung zum Anbringen der Beduftungsvorrichtung im Innenraum des Fahrzeuges, wobei die Haltevorrichtung an der Rückseite des Abdeckelements befestigt ist. Die Begriffe "Vorderseite" und "Rückseite" definieren sich somit durch die Position der Haltevorrichtung. Die "Rückseite" kann nur diejenige Seite sein, an welcher die Haltevorrichtung angeordnet ist. Insbesondere ist an der Vorderseite keine Haltevorrichtung angeordnet. Insbesondere ist die Vorderseite diejenige Seite, welche von derjenigen Fläche im Innenraum des Fahrzeuges, an welchem die Beduftungsvorrichtung zur Montage vorgesehen ist, abgewendet ist. Anstelle von "Vorderseite" und "Rückseite" könnte auch von "einer ersten Seite" und "einer zweiten Seite" gesprochen werden.
- einen Duftstoffbehälter, welcher rückseitig zum Abdeckelement angeordnet ist und am Abdeckelement und/oder an der Haltevorrichtung befestigt ist. "Rückseitig" bedeutet auf derjenigen Seite des Abdeckelements, auf welcher auch die Haltevorrichtung angeordnet ist. Damit verdeckt das Abdeckelement den Duftstoffbehälter zumindest teilweise bei Betrachtung von der Vorderseite.
Der Duftstoffbehälter besteht insbesondere aus einem Kompositwerkstoff mit zumindest einem Polymer, insbesondere einem Elastomer, und dem Duftstoff. Dabei ist insbesondere das Polymer mit dem Duftstoff angereichert und fungiert als dessen Träger.

Die Vorderseite des Abdeckelements weist eine grössere Fläche auf als eine Aussenfläche des Duftstoffbehälters, welche gegen die Rückseite des Abdeckelements gerichtet ist. Dieser Flächenvergleich bedeutet, dass das Abdeckelement den Duftstoffbehälter hinter sich verbergen kann, sofern die Beduftungsvorrichtung frontal von vorne betrachtet wird und der Duftstoffbehälter hinter dem Abdeckelement positioniert ist. Eine solche Beduftungsvorrichtung ermöglicht, den Duftstoffbehälter möglichst diskret oder optisch ansprechend im Innenraum eines Fahrzeuges anzuordnen. Nicht die Optik des Duftstoffbehälters, sondern die Optik des Abdeckelements wird von einem Fahrzeuginsassen wahrgenommen.

Insbesondere umfasst das Abdeckelement keine Öffnungen. Dies hat zur Folge, dass der hinter dem Abdeckelement angeordnete Duftstoffbehälter mit Sicht von vorne überhaupt nicht sichtbar ist.

Mit Vorteil umfasst der Duftstoffbehälter Öffnungen zur Durchströmung von Luft. Dies ermöglicht, dass Luft durch den Duftstoffbehälter strömen und Duftmoleküle mitreissen kann, um eine angenehme Raumluft im Innenraum des Fahrzeuges zu gewährleisten.

Vorteilhaft umfasst die Haltevorrichtung ein Befestigungselement zum Befestigen der Beduftungsvorrichtung im Fahrzeug. Das Befestigungselement umfasst eine Klebeschicht oder ein Saugelement oder ein Klettverschlusselement. Dadurch wird ermöglicht, dass die Beduftungsvorrichtung an möglichst unterschiedlichen Orten im Innenraum des Fahrzeuges montiert werden kann. Insbesondere kann je nach Befestigung die Beduftungsvorrichtung rückstandsfrei demontiert werden.

In einer besonderen Ausführungsform ist das Abdeckelement plattenförmig ausgestaltet. "Plattenförmig" bedeutet, dass die Vorderseite als eine ebene oder allenfalls leicht gekrümmte Fläche geformt ist. Zudem sind die Vorderseite und die Rückseite des Abdeckelements viel grösser als die übrigen Seitenflächen des Abdeckelements ausgestaltet. Insbesondere sind die Länge und die Breite des Abdeckelements viel grösser als die Dicke des Abdeckelements.

Mit Vorteil verdeckt das Abdeckelement den Duftstoffbehälter vollständig oder zumindest grösstenteils, d.h. insbesondere zu mindestens 70%, insbesondere zu mindestens 80%, sofern mit Blick von vorne, d.h. in Normalenrichtung zur Vorderseite des Abdeckelements, auf das Abdeckelement geschaut wird. Der Duftstoffbehälter ist damit für einen Insassen im Fahrzeuginnenraum kaum oder gar nicht sichtbar.

Vorteilhaft weist zumindest die äusserste Schicht an der Vorderseite des Abdeckelements eine Glasoptik auf. Insbesondere besteht die äusserste Schicht aus Glas oder Acrylglas, oder aus einem optisch glasähnlichen Material. Dies ermöglicht, dass die Beduftungsvorrichtung in der Nähe eines Displays im Innenraum des Fahrzeuges möglichst diskret montiert werden kann, da die sichtbare Oberfläche der Beduftungsvorrichtung eine ähnliche Optik wie ein Display aufweist. Insbesondere ist die äusserste Schicht des Abdeckelements mit einer schwarzen Folie hinterlegt.

Mit Vorteil ist das gesamte Abdeckelement in Richtung von der Vorderseite zur Rückseite nicht transparent. Dadurch ist der hinter dem Abdeckelement angeordnete Duftstoffbehälter mit Sicht von vorne nicht sichtbar.

Insbesondere ist der Duftstoffbehälter mittels einer Koppelvorrichtung am Abdeckelement und/oder an der Haltevorrichtung befestigt, wobei die Koppelvorrichtung zum wiederholten Koppeln und entkoppeln, insbesondere ohne Einsatz von Werkzeugen, geeignet ist. Dies hat den Vorteil, dass der Benutzer den Duftstoffbehälter auswechseln, das Abdeckelement jedoch wiederverwenden kann.

Mit Vorteil ist die Koppelvorrichtung ausgestaltet, mittels eines Formschlusses oder mittels eines Kraftschlusses zu koppeln. Insbesondere weist die Koppelvorrichtung einen Klemmverschluss oder einen Schnappverschluss auf.

Alternativ weist die Koppelvorrichtung ein Federelement auf, dessen Federkraft zum Koppeln und Entkoppeln überwunden werden muss.

Im Weiteren stellt sich die Aufgabe, ein Fahrzeug bereitzustellen, wobei der Duftstoffbehälter möglichst unauffällig im Innenraum eines Fahrzeugs angeordnet ist.

Die Aufgabe wird durch das Fahrzeug mit den Merkmalen des Anspruchs 11 gelöst. Das Fahrzeug umfasst eine im Innenraum des Fahrzeuges angeordnete Paneele mit mindestens zwei Displays. Insbesondere befinden sich die mindestens zwei Displays in einem Bereich des Innenraums, welcher durch den Fahrzeugführer mit seinen Händen gut erreichbar ist, um das Display zu berühren. Insbesondere ist mindestens eines der mindestens zwei Displays als ein berührungsempfindliches Display ausgestaltet.

Zudem umfasst das Fahrzeug eine Beduftungsvorrichtung mit einem Duftstoffbehälter und mit einem Abdeckelement zum zumindest teilweisen Abdecken des Duftstoffbehälters. Die Beduftungsvorrichtung ist zwischen den mindestens zwei Displays an die Paneele befestigt.

Die Anordnung der Beduftungsvorrichtung an die Paneele zwischen den zwei Displays hat den Vorteil, dass die Beduftungsvorrichtung optisch diskret und ohne Beeinträchtigung der Displays montiert ist.

Insbesondere ist die Beduftungsvorrichtung mittels Adhäsion, insbesondere mittels Klebung, mittels eines Klettverschlusses oder mittels eines Saugelements an die Paneele befestigt. Diese Verbindungstechnologien erlauben es besonders gut, die Beduftungsvorrichtung an die Paneele zu befestigen.

In einer besonderen Ausführungsform weist das Abdeckelement an seiner Vorderseite eine Aussenschicht auf, welche optisch äquivalent zum mindestens einen Display im deaktivierten Zustand ist, insbesondere wobei das Abdeckelement an seiner Vorderseite eine Schicht aus einem identischen Material aufweist, wie die Aussenschicht des mindestens einen Displays und/oder wie die Aussenschicht der Paneele in demjenigen Bereich, in welchem die Beduftungsvorrichtung befestigt ist. Dies ermöglicht, dass die Beduftungsvorrichtung optisch unauffällig am Display angeordnet werden kann.

Mit Vorteil weist die Paneele im Bereich, in welchem die Beduftungsvorrichtung befestigt ist, insbesondere auf der gesamten Paneele, eine Aussenschicht aus einem gläsernen Material, insbesondere aus Acrylglas, auf. Insbesondere ist die Aussenschicht der Paneele im besagten Bereich, insbesondere auf der gesamten Paneele, mit einer schwarzen Folie hinterlegt.

Insbesondere ist die Beduftungsvorrichtung derart ausgestaltet und an die Paneele befestigt, dass der Duftstoffbehälter ausgetauscht werden kann, ohne das Abdeckelement zu demontieren. Dies hat den Vorteil, dass die Beduftungsvorrichtung stets mit der Paneele verbunden bleibt und ein ständiges Koppeln und Entkoppeln die Koppelvorrichtung nicht beschädigt.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1a den Innenraum eines Fahrzeuges mit Blick von der hinteren Sitzreihe nach vorne, wobei eine erfindungsgemässe Beduftungsvorrichtung an einer Paneele angeordnet ist;
Fig. 1b die Paneele des Fahrzeuges gemäss Fig. 1a in Detailansicht;
Fig. 2 die erfindungsgemäss Beduftungsvorrichtung mit Blick von vorne;
Fig. 3 die erfindungsgemässe Beduftungsvorrichtung mit Blick von oben;
Fig. 4 die erfindungsgemässe Beduftungsvorrichtung in einer Schnittansicht gemäss der in Fig. 3 gezeigten Schnittlinie A-A; und
Fig. 5 die erfindungsgemässe Beduftungsvorrichtung in einer weiteren Schnittansicht gemäss der in Fig. 3 gezeigten Schnittlinie B-B.

### Weg zur Ausführung der Erfindung

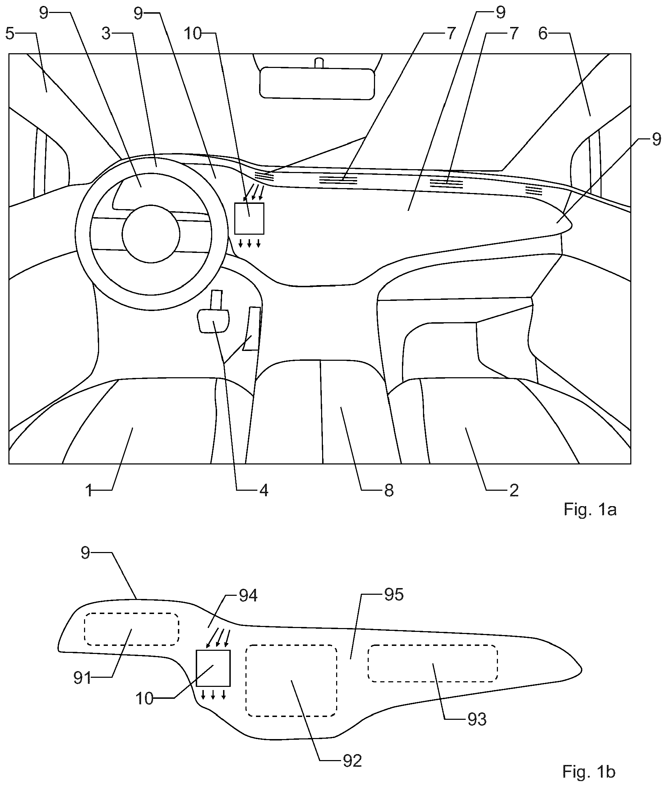

Die Fig. 1a zeigt denn Innenraum eines Personenkraftfahrzeuges mit Blick von der Mitte der zweiten Sitzreihe nach vorne. Der Innenraum umfasst einen Fahrersitz 1, einen Beifahrersitz 2, ein Lenkrad 3, Gas- und Bremspedale 4, zwei A-Säulen 5 und 6, Lüftungsschlitze 7, eine Mittelkonsole 8 und eine Paneele 9 mit drei Displays 91, 92, 93.

Die Paneele 9 erstreckt sich von der linken A-Säule 5 bis zur rechten A-Säule 6. Wie in Fig. 1b gezeigt, umfasst die Paneele 9 insgesamt drei Displays 91, 92 und 93. Die drei Displays 91, 92 und 93 sind mit gestrichelten Umrahmungen in Fig. 1b illustriert. Der linke Display 91 stellt die Cockpitanzeige dar und zeigt dem Fahrzeugführer wichtige Fahrinformationen, wie z.B. Geschwindigkeit, Energiestatus, Navigationsinformationen oder Warnungen, an. Im mittleren Display 92 können verschiedenste Apps aufgerufen werden, beispielsweise ein Autoradio, Klimaeinstellungen, Telefonie, allgemeine Fahrzeugeinstellungen oder das Fahrzeughandbuch. Das rechte Display 93 kann beispielsweise der Unterhaltung des Beifahrers dienen, beispielsweise durch Anzeige von Videos. Die drei Displays 91, 92 und 93, oder nur ein eines oder nur zwei der Displays können als berührungsempfindliches Display ausgestaltet sein. Beispielsweise ist nur das mittlere Display 92 berührungsempfindlich ausgestaltet, während das linke Display 91 und das rechte Display 93 ausschliesslich der Anzeige von Inhalten dienen. Das mittlere Display 92 kann sowohl vom Fahrzeugführer, als auch vom Beifahrer zur Berührbedienung erreicht werden.

Zwischen den einzelnen Displays 91, 92, 93 weist die Paneele 9 Zwischenräume 94 und 95 auf. Die Zwischenräume 94 und 95 weisen eine gleiche Oberflächenoptik wie die Displays 91, 92, 93 in deaktiviertem Zustand auf. Im linken Zwischenraum 94 ist beispielsweise kein Display angeordnet, weil dieser Bereich weder vom Fahrzeugführer noch vom Beifahrer gut sichtbar ist. Das Lenkrad 3 verdeckt die Sicht für den Fahrer und für den Beifahrer ist der linke Zwischenraum 94 zu weit entfernt, um diesen gut wahrnehmen zu können.

An der Paneele 9 ist im Bereich des linken Zwischenraums 94 eine Beduftungsvorrichtung 10 angeordnet. Die Beduftungsvorrichtung 10 umfasst einen Duftstoff, welcher von der im Innenraum des Fahrzeuges zirkulierenden Luft beim Durchströmen der Beduftungsvorrichtung 10 mitgerissen und im Innenraum verteilt wird. Bei der vorliegenden Positionierung der Beduftungsvorrichtung 10 an der Paneele 9 strömt Luft aus dem oberhalb der Beduftungsvorrichtung 10 angeordneten Lüftungsschlitz 7 nach unten durch die Beduftungsvorrichtung 10. Die Luftströmung ist mittels Pfeilen illustriert.

Die gesamte Paneele 9 mit den drei Displays 91, 92 und 92 weist eine Aussenschicht auf, welche aus Acrylglas besteht. Hinter der Aussenschicht der Paneele 9 ist eine schwarze Folie hinterlegt. Da das Acrylglas transparent ist, lässt die schwarze Folie die Paneele in schwarzer Farbe erscheinen.

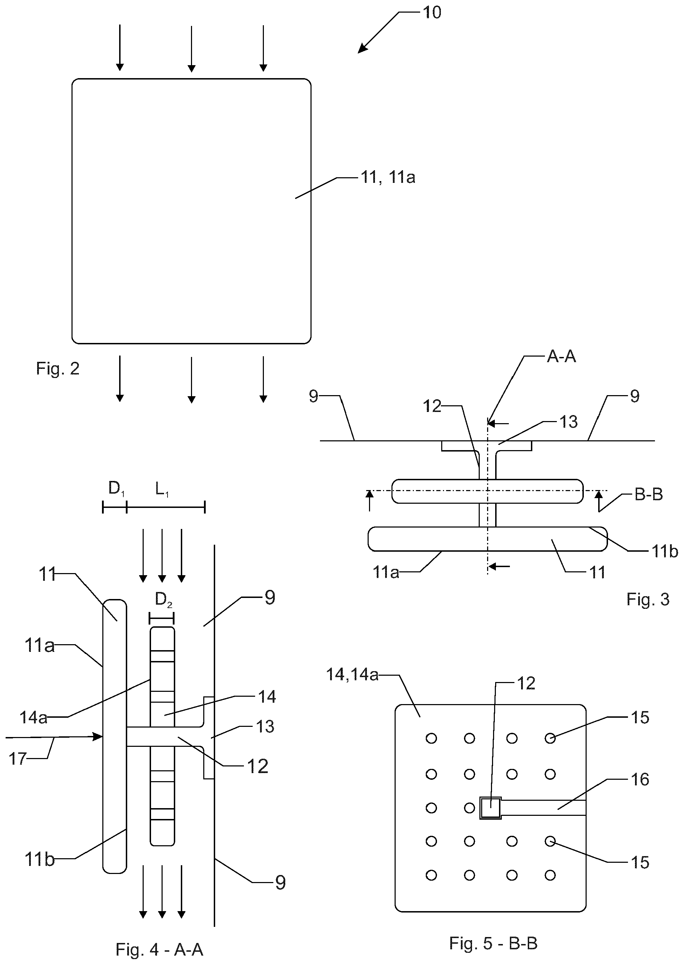

Die Fig. 2, 3, 4 und 5 zeigen die Beduftungsvorrichtung 10 in detaillierter Ansicht. Die Fig. 2 zeigt die Beduftungsvorrichtung 10 mit Blick von vorne, d.h. in gleicher Perspektive wie in den Fig. 1a und 1b. Die Fig. 3 zeigt die Beduftungsvorrichtung 10 von oben, d.h. aus derjenigen Richtung aus welcher die Luft in die Beduftungsvorrichtung 10 aus dem Lüftungsschlitz 7 hineinströmt. Die Fig. 4 zeigt einen Schnitt durch die Beduftungsvorrichtung 10. Die Schnittlinie und die Blickrichtung sind in Fig. 3 illustriert und mit dem Bezugszeichen A-A markiert. Die Fig. 5 zeigt einen weiteren Schnitt, wobei die Schnittlinie in Fig. 3 mit dem Bezugszeichen B-B markiert ist. Der Duftstoffbehälter ist mit Blick von vorne dargestellt, d.h. gleich wie in Fig. 2, wobei der Duftstoffbehälter in Fig. 2 verdeckt ist.

Die Beduftungsvorrichtung 10 umfasst ein plattenförmiges Abdeckelement 11 mit einer Vorderseite 11a und einer Rückseite 11b. Das Abdeckelement 11 ist 72 mm hoch und 50 mm breit. Seine Dicke D₁ beträgt 5 mm. Die Vorderseite 11a weist eine Glasoptik auf, welche der Optik eines deaktivierten Displays 9 entspricht. Hierfür besteht die Oberfläche der Vorderseite 11a aus Acrylglas mit einer schwarzen Folie, welche hinter dem Acrylglas angeordnet ist. Das Abdeckelement 11 ist in Richtung seiner Dicke D₁ nicht transparent und weist auch keine Öffnungen auf.

An dem gegen die Paneele 9 gerichteten Ende der Haltevorrichtung 12 weist die Haltevorrichtung 12 ein Befestigungselement 13 mit einer Klebeschicht auf. Die Klebeschicht verbindet die Beduftungsvorrichtung mit der Paneele 9. Die Klebeschicht ist derart ausgestaltet, dass die Beduftungsvorrichtung rückstandsfrei von der Paneele entfernt werden kann.

Im Weiteren umfasst die Beduftungsvorrichtung 10 eine Haltevorrichtung 12, mittels welcher die Beduftungsvorrichtung 10 an der Paneele 9 befestigt ist. Die Haltevorrichtung 12 weist einen quadratischen Querschnitt auf, wie es in Fig. 5 gut sichtbar ist. Die Haltevorrichtung 12 ist an einem Ende zentrisch mit dem Abdeckelement 11 verbunden. An ihrem anderen Ende ist die Haltevorrichtung 12 an der Paneele 9 befestigt. Die Haltevorrichtung 12 weist eine Länge L₁ von 15 mm auf.

An der Haltevorrichtung 12 ist ein Duftstoffbehälter 14 angeordnet. Der Duftstoffbehälter 14 ist zwischen dem Abdeckelement 11 und der Paneele 9 angeordnet. Die Vorderseite 11a des Abdeckelements 11 weist eine Fläche auf, welche grösser ist, als die Aussenfläche 14a des Duftstoffbehälters, welche gegen die Rückseite 11b des Abdeckelements 11 gerichtet ist. Dieses Flächenverhältnis sorgt dafür, dass mit Blick von vorne auf die Vorderseite 11a des Abdeckelements 11 das Abdeckelement 11 den Duftstoffbehälter 14 verdeckt. D.h. mit Betrachtung in Normalenrichtung 17 zur Vorderseite 11a wird der Duftstoffbehälter 14 vom Abdeckelement 11 verdeckt. Der Duftstoffbehälter 14 weist eine Dicke D₂ von 5 mm auf. Der Duftstoffbehälter 14 ist sowohl zum Abdeckelement 11, als auch zur Paneele 9 jeweils 5 mm beabstandet.

Der Duftstoffbehälter 14 ist aus einen Kompositwerkstoff aus zumindest einem Polymer, insbesondere einem Elastomer, gefertigt und mit dem Duftstoff angereichert. D.h. der Kompositwerkstoff fungiert als Träger des Duftstoffs. Der Duftstoffbehälter 14 weist mehrere Durchlassöffnungen 15 auf, durch welche die aus dem Lüftungsschlitz 7 austretende Luft strömen kann. Zudem ist die Aussenseite des Duftstoffbehälters 14 leicht gewellt, was in den Zeichnungen jedoch nicht weiter illustriert ist. Durch das Vorsehen der Durchlassöffnungen 15 und einer gewellten Oberfläche wird die Oberfläche des Duftstoffbehälters 14 vergrössert, an welcher die aus dem Lüftungsschlitz austretende Luft mit dem Duftstoff in Kontakt geraten kann. Dies hat eine erhöhte Anreicherung der den Duftstoffbehälter 14 durchströmenden Luft mit Duftstoffmolekülen zur Folge. Alternativ kann der Duftstoffbehälter 14 auch in Form eines Granulats vorgesehen sein, das in einem Käfig enthalten ist.

Der Duftstoffbehälter 14 weist einen Spalt 16 auf, welcher von einer Aussenseite des Duftstoffbehälter 14 bis in die Mitte des Duftstoffbehälters 14 führt. Der Spalt 16 ermöglicht, dass der Duftstoffbehälter 14 über die Haltevorrichtung 12 geschoben werden kann bis die Haltevorrichtung 12 in der Mitte des Duftstoffbehälters 14 zu liegen kommt. Das Querschnittprofil des Spalts 16 ist leicht kleiner als das Querschnittprofil der Haltevorrichtung 12, sodass für das Einführen der Haltevorrichtung 12 in den Spalt 16 ein gewisser Kraftaufwand erforderlich ist. Der Duftstoffbehälter 14 ist somit mittels eines Formschlusses an der Haltevorrichtung 12 gehalten, da der verengte Spalt 16 die Bewegung der Haltevorrichtung 12 von der Mittelposition in den Spalt 16 erschwert. Der Formschluss soll verhindern, dass wenn der Duftstoffbehälter 14 in Position ist, nicht leicht von der Haltevorrichtung 12 gelöst werden kann. Das Querschnittsprofil des Spalts 16 stellt gleichzeitig auch ein Federelement dar, welchem entgegengewirkt werden muss, um den Duftstoffbehälter 14 auf der Haltevorrichtung 12 zu montieren oder von dieser zu entfernen. Das quadratisch geformte Querschnittprofil der Haltevorrichtung 12 verhindert, dass sich der Duftstoffbehälter 14 um die Haltevorrichtung 12 dreht.

Der Spalt 16 stellt eine Koppelvorrichtung dar, welche die Kopplung des Duftstoffbehälters 14 an die Haltevorrichtung 12 ermöglicht. Der Duftstoffbehälter 14 kann wiederholt mit der Haltevorrichtung 12 gekoppelt und entkoppelt werden, ohne dass hierfür Werkzeuge benötigt würden. Vorteilhaft ist auch, dass der Duftstoffbehälter 14 an die Beduftungsvorrichtung gekoppelt oder von dieser entkoppelt werden kann, ohne dass das Abdeckelement 11 entfernt werden müsste.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Beduftungsvorrichtung (10) für ein Fahrzeug aufweisend
- ein Abdeckelement (11) mit einer Vorderseite (11a) und einer Rückseite (11b),
- eine Haltevorrichtung (12) zum Anbringen der Beduftungsvorrichtung (10) im Innenraum des Fahrzeuges, wobei die Haltevorrichtung (12) an der Rückseite (11b) des Abdeckelements (11) befestigt ist,
- einen Duftstoffbehälter (14), welcher rückseitig zum Abdeckelement (11) angeordnet ist und am Abdeckelement (11) und/oder an der Haltevorrichtung (12) befestigt ist,
**dadurch gekennzeichnet, dass** die Vorderseite (11a) des Abdeckelements (11) eine grössere Fläche aufweist als eine Aussenfläche (14a) des Duftstoffbehälters (14), welche gegen die Rückseite (11b) des Abdeckelements (11) gerichtet ist.

2. Beduftungsvorrichtung nach Anspruch 1, wobei das Abdeckelement (11) keine Öffnungen aufweist.

3. Beduftungsvorrichtung nach einem der vorangehenden Ansprüche, wobei der Duftstoffbehälter (14) Öffnungen (15) zur Durchströmung von Luft aufweist.

4. Beduftungsvorrichtung nach einem der vorangehenden Ansprüche, wobei die Haltevorrichtung (12) ein Befestigungselement (13) zum Befestigen der Beduftungsvorrichtung (10) im Fahrzeug aufweist, wobei das Befestigungselement (13) eine Klebeschicht oder ein Saugelement oder ein Klettverschlusselement aufweist.

5. Beduftungsvorrichtung nach einem der vorangehenden Ansprüche, wobei das Abdeckelement (11) plattenförmig ausgestaltet ist.

6. Beduftungsvorrichtung nach Anspruch 5, wobei mit Betrachtung in Normalenrichtung (17) zur Vorderseite (11a) des Abdeckelements (11) das Abdeckelement (11) den Duftstoffbehälter (14) vollständig oder zumindest grösstenteils verdeckt.

7. Beduftungsvorrichtung nach einem der vorangehenden Ansprüche, wobei zumindest die äusserste Schicht an der Vorderseite (11a) des Abdeckelements (11) aus Glas, insbesondere aus Acrylglas, oder aus einem optisch glasähnlichen Material besteht,
insbesondere wobei die äusserste Schicht von einer schwarzen Folie hinterlegt ist.

8. Beduftungsvorrichtung nach einem der vorangehenden Ansprüche, wobei das gesamte Abdeckelement (11) in Richtung von der Vorderseite (11a) zur Rückseite (11b) nicht transparent ist.

9. Beduftungsvorrichtung nach einem der vorangehenden Ansprüche, wobei der Duftstoffbehälter (14) mittels einer Koppelvorrichtung (16) am Abdeckelement (14) und/oder an der Haltevorrichtung (12) befestigt ist, wobei die Koppelvorrichtung (16) zum wiederholten Koppeln und entkoppeln, insbesondere ohne Einsatz von Werkzeugen, geeignet ist.

10. Beduftungsvorrichtung nach Anspruch 9, wobei die Koppelvorrichtung (14) ausgestaltet ist,
- mittels eines Formschlusses oder mittels eines Kraftschlusses zu koppeln, oder
- ein Federelement aufweist, dessen Federkraft zum Koppeln und Entkoppeln überwunden werden muss.

11. Fahrzeug, aufweisend
- eine im Innenraum des Fahrzeuges angeordnete Paneele (9) mit mindestens zwei Displays (91,92,93),
- eine Beduftungsvorrichtung (10) mit einem Duftstoffbehälter (14) und einem Abdeckelement (11) zum zumindest teilweisen Abdecken des Duftstoffbehälters (14),
**dadurch gekennzeichnet, dass** die Beduftungsvorrichtung (10) zwischen den mindestens zwei Displays (91,92,93) an die Paneele (9) befestigt ist,
insbesondere wobei die Beduftungsvorrichtung (10) nicht an einen Lüftungsauslass (7) befestigt ist.

12. Fahrzeug nach Anspruch 11, wobei die Beduftungsvorrichtung (10) mittels Adhäsion, insbesondere mittels Klebung, mittels eines Klettverschlusses oder mittels eines Saugelements an die Paneele (9) befestigt ist.

13. Fahrzeug nach einem der Ansprüche 11 bis 12, wobei die Beduftungsvorrichtung (10) nach einem der Ansprüche 1 bis 10 ausgestaltet ist.

14. Fahrzeug nach einem der Ansprüche 11 bis 13, wobei das Abdeckelement (11) an seiner Vorderseite (11a) eine Aussenschicht aufweist, welche optisch äquivalent ist zum mindestens einen Display (91,92,93) im deaktivierten Zustand oder zur Aussenschicht der Paneele (9) in demjenigen Bereich (94), in welchem die Beduftungsvorrichtung (10) befestigt ist,
insbesondere wobei das Abdeckelement (91,92,93) an seiner Vorderseite (11a) eine Schicht aus einem identischen Material aufweist, wie die Aussenschicht des mindestens einen Displays (91,92,93) und/oder wie die Aussenschicht der Paneele (9) in demjenigen Bereich (94), in welchem die Beduftungsvorrichtung (10) befestigt ist.

15. Fahrzeug nach einem der Ansprüche 11 bis 14, wobei die Paneele (9) im Bereich (94), in welchem die Beduftungsvorrichtung (10) befestigt ist, insbesondere auf der gesamten Paneele (9), eine Aussenschicht aus einem gläsernen Material, insbesondere aus Acrylglas, aufweist,
insbesondere wobei die Aussenschicht der Paneele (9) im besagten Bereich (94), insbesondere auf der gesamten Paneele (9), mit einer schwarzen Folie hinterlegt ist.

16. Fahrzeug nach einem der Ansprüche 11 bis 15, wobei die Beduftungsvorrichtung (10) derart ausgestaltet und an die Paneele (9) befestigt ist, dass der Duftstoffbehälter (14) ausgetauscht werden kann, ohne das Abdeckelement (11) zu demontieren.
